(19) Europäisches Patentamt / European Patent Office / Office européen des brevets

(11) EP 3 471 837 B1

(12) **FASCICULE DE BREVET EUROPEEN**

(45) Date de publication et mention de la délivrance du brevet:
**12.08.2020 Bulletin 2020/33**

(21) Numéro de dépôt: **17729164.8**

(22) Date de dépôt: **14.06.2017**

(51) Int Cl.:
*A61Q 19/08* (2006.01) *C12N 5/00* (2006.01)
*A61K 36/48* (2006.01) *A61K 31/4409* (2006.01)
*A61K 8/9789* (2017.01) *A61P 17/04* (2006.01)
*A61P 17/06* (2006.01) *A61P 17/18* (2006.01)

(86) Numéro de dépôt international:
**PCT/EP2017/064640**

(87) Numéro de publication internationale:
**WO 2017/216274 (21.12.2017 Gazette 2017/51)**

(54) **EXTRAIT DE CELLULES INDIFFERENCIEES DE MIMOSA PUDICA ET SES UTILISATIONS EN DERMO-COSMETIQUE**

EXTRAKT AUS UNDIFFERENZIERTEN ZELLEN VON MIMOSA PUDICA UND VERWENDUNGEN DAVON IN DERMOKOSMETIK

EXTRACT OF UNDIFFERENTIATED CELLS OF MIMOSA PUDICA AND USES THEREOF IN DERMO-COSMETICS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.06.2016 FR 1655630**

(43) Date de publication de la demande:
**24.04.2019 Bulletin 2019/17**

(73) Titulaire: **Pierre Fabre Dermo-Cosmétique**
**92100 Boulogne-Billancourt (FR)**

(72) Inventeurs:
- **NGUYEN, Thien**
  **31180 Rouffiac-Tolosan (FR)**
- **COUSY, Adrien**
  **31870 Beaumont sur Leze (FR)**

(74) Mandataire: **Regimbeau**
**20, rue de Chazelles**
**75847 Paris Cedex 17 (FR)**

(56) Documents cités:
**FR-A1- 2 864 446 US-B1- 6 290 993**

- **Neeraj K Patel ET AL: "Original article: SUPPRESSIVE EFFECTS OF MIMOSA PUDICA (L.) CONSTITUENTS ON THE PRODUCTION OF LPS-INDUCED PRO-INFLAMMATORY MEDIATORS", EXCLI Journal, 24 juillet 2014 (2014-07-24), pages 1011-1021, XP055346557, Extrait de l'Internet: URL:https://www.ncbi.nlm.nih.gov/pmc/articles/PMC4464187/pdf/EXCLI-13-1011.pdf [extrait le 2017-09-15]**
- **SUGIYAMA MUNETAKA: "Historical review of research on plant cell dedifferentiation", JOURNAL OF PLANT RESEARCH, TOKYO, JP, vol. 128, no. 3, 1 mars 2015 (2015-03-01), pages 349-359, XP035491567, ISSN: 0918-9440, DOI: 10.1007/S10265-015-0706-Y [extrait le 2015-03-01]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention concerne une préparation issue d'une culture in vitro de cellules indifférenciées de Mimosa pudica ainsi que son procédé de préparation; une composition cosmétique ou dermatologique comprenant ladite préparation ; et ses utilisations pour le traitement des troubles inflammatoires cutanés, comme agent anti-oxydant dont le traitement des stress oxydatifs dus à la pollution environnementale, et comme agent anti-âge.

Mimosa pudica est une plante médicinale utilisée en Asie depuis plusieurs siècles pour soigner l'inflammation. Mimosa pudica Linn. (Fabaceae) est connu pour ses vertus antidiabétiques (Maries 1995), antidépressive (Molina 1999), anti-inflammatoire (Patel 2014), antioxydant (Patro 2016), antibactérien (Bhakuni 1969). Elle est utilisée également dans la cicatrisation des plaies (Paul et al., 2010. Int J Bio Med Res 1(4) :223-227).

L'extrait méthanolique des feuilles de Mimosa pudica contient des molécules actives telles que: terpènes, flavonoïdes, glycosides, alcaloïdes, quinines, phénols, tannins, saponins et coumarin (Gandhiraja et al. 2009). Les molécules phénoliques contenues expriment une forte activité anti-oxydante (Zhang et al. 2011. Pharmacogn. Mag 4 :35-39). Cependant, une molécule majeure isolée de la plante, est très remarquée par sa toxicité : la mimosine. Il s'agit un acide aminé non protéique (beta-N(3-hydroxy-4-pyridone)-alpha-amino propionic acid). Il a été montré que la mimosine induisait chez l'animal des effets indésirables tels que: perte d'appétit, perte de cheveux, troubles de reproduction (Kulp K.S. 1996. Toxicology and applied pharmacology.139 :356-364). Il a également été démontré qu'elle inhibait la germination des graines ou la synthèse de l'ADN des cellules mises en culture (Williams RD et al., 2007. Allelopathy J. 19(2) :423-430 ; Stuenzi et al., 1979). Il semble que la mimosine soit synthétisée par la plante afin de se défendre contre les ruminants qui broutent la plante : elle provoque des troubles digestifs chez l'animal. La présence de mimosine dans les extraits de Mimosa pudica est un facteur limitant pour la valorisation de cette plante médicinale. Une approche consisterait à trouver des moyens de purification pour l'éliminer, mais cette voie alourdit les procédés et nécessite un contrôle accru et par conséquent un coût de revient plus élevé.

La littérature est abondante pour les préparations d'extraits avec des solvants polaires et non polaires. Le document KR 10-1064848 décrit des extraits à partir de la plante par des solvants organiques tels: l'éthanol, l'acétate d'éthyle, pour le traitement des maladies auto-immunes.

Dans le cadre de la présente invention, la Demanderesse a mis en évidence une nouvelle valorisation de Mimosa pudica via une voie alternative : la culture in vitro de cellules indifférenciées, totipotentes. En effet, il a été remarqué de manière surprenante que la culture de ces cellules en bioréacteur permet d'obtenir une préparation significativement appauvrie en mimosine ; ladite préparation présentant des propriétés très intéressantes pour le traitement des troubles inflammatoires cutanés, des stress oxydatifs, et du vieillissement cutané. Ces propriétés ont été plus particulièrement surprenantes lorsqu'une étape de bioconversion est réalisée en cours de procédé, cette étape conduisant à l'obtention de nouveaux métabolites qui potentialisent plus particulièrement l'activité anti-oxydante. De manière inattendue, la Demanderesse a constaté que ces métabolites font partie des N-phenylpropenoyl-L amino acid (NPA). Les NPA en tant que tels sont connus et très recherchés pour leur activité pharmacologique intéressante (Hensel et al. Planta Med. 2007; 73: 142 - 150; Zeng et al. J. Agric. Food Chem. 2011 ; 59: 5342-5350). A ce jour, il n'était pas connu que la plante Mimosa pudica contienne des anti-oxydants comme les NPA.

Dans les dermatoses inflammatoires, dans les lésions de la peau des patients atteints de la dermatite atopique au niveau de l'épiderme, on observe une surexpression de cytokine TSLP (Thymic stromal lymphopoietin) qui joue un rôle crucial dans la pathogénèse des maladies allergiques médiée par une réponse cellulaire type Th2 (Takai et al. 2012. Allergology Int. 61 :3-17). Le TSLP est décrit comme responsable du prurit souvent présent en dermatite atopique ou dans le psoriasis. Nous démontrons pour la première fois qu'un extrait de culture de cellules végétales (CCV) de Mimosa pudica obtenu par le procédé selon l'invention présente des activités anti-oxydantes et anti-inflammatoires et montre notamment une inhibition très forte de TSLP dans le modèle in vitro de la dermatite atopique. Nous démontrons également qu'en absence de la Mimosine dans nos extraits, un extrait de CCV de Mimosa pudica selon l'invention conserve l'activité pharmacologique anti-inflammatoire.

Le procédé d'obtention de la préparation, objet de la présente invention, consiste en la dédifférenciation cellulaire à partir de matériel végétal issu de Mimosa pudica, puis en la culture de cellules en suspension à l'état indifférencié afin d'obtenir rapidement une biomasse fine, abondante, homogène et stérile de cette plante. La culture cellulaire rend modulable les voies de biosynthèse de cette plante à l'échelle cellulaire.

La présente invention vise donc une préparation issue d'une culture in vitro de cellules indifférenciées de Mimosa pudica, une composition cosmétique ou dermatologique comprenant ladite préparation et ses utilisations en cosmétologie et/ou en dermatologie et plus préférentiellement pour le traitement des troubles inflammatoires cutanés, comme agent anti-oxydant dont le traitement des stress oxydatifs dus à la pollution environnementale (tabac, air pollué intérieur et extérieur par des agents chimiques ou des allergènes), et comme agent anti-âge.

C'est ainsi que la présente invention concerne un procédé de préparation in vitro d'un extrait cellulaire de Mimosa pudica présentant une teneur en mimosine inférieure à 5 ng/g de matière sèche comprenant les étapes suivantes :

- a. Fourniture de matériel végétal stérile de Mimosa pudica,
- b. Dédifférenciation des cellules du matériel végétal,
- c. Mise en culture en suspension des cellules indifférenciées dans un milieu liquide permettant leur maintien dans l'état indifférencié,
- d. Culture de propagation d'une biomasse de cellules indifférenciées dans le milieu de culture,
- e. arrêt de la propagation et obtention d'un extrait cellulaire présentant une teneur en mimosine inférieure à 5 ng/g de matière sèche.

Dans un mode particulier de réalisation, le procédé selon l'invention est caractérisé en ce que le matériel végétal de Mimosa pudica est choisi dans le groupe constitué par feuille, tige, pétiole, racine, graine, fleur et bourgeon. Dans un mode particulier de réalisation, le procédé selon l'invention est caractérisé en ce que l'étape b) de dédifférenciation est réalisée sur un milieu solide comprenant un ou plusieurs facteurs de croissance.
Dans un mode particulier de réalisation, le procédé selon l'invention est caractérisée en ce que le un ou plusieurs facteurs de croissance comprend une hormone choisie dans le groupe constituée par les auxines, les cytokines, les gibbérellines et leurs mélanges.
Dans un mode particulier de réalisation, le procédé selon l'invention est caractérisé en ce que l'étape b) de dédifférenciation est répétée permettant l'obtention de cals de cellules dédifférenciés.
Dans un mode particulier de réalisation, le procédé selon l'invention est caractérisé en ce que l'étape c) est réalisée dans un milieu liquide comprenant un ou plusieurs facteurs de croissance.
Dans un mode particulier de réalisation, le procédé selon l'invention est caractérisé en ce que le ou les facteurs de croissance sont les mêmes que celui ou ceux du milieu de dédifférenciation.
Dans un mode particulier de réalisation, le procédé selon l'invention est caractérisé en ce que l'étape d) de culture de propagation est réalisée par des repiquages ou dilutions successifs dans le milieu de culture liquide jusqu'à obtention d'une densité de cellules constante.
Dans un mode particulier de réalisation, le procédé selon l'invention est caractérisé en ce qu'il comprend les étapes supplémentaires suivantes :

- f. séparation liquide/solide,
- g. récupération d'un extrait cellulaire consistant en la biomasse séparée du milieu de culture présentant une teneur en mimosine inférieure à 5 ng/g de matière sèche.

Dans un autre mode particulier de réalisation, le procédé selon l'invention est caractérisé en ce qu'il comprend les étapes supplémentaires suivantes :

- f. séparation liquide/solide,
- g. récupération de l'extrait cellulaire consistant en la phase liquide de milieu de culture présentant une teneur en mimosine inférieure à 5 ng/g de matière sèche.

Dans un mode particulier de réalisation, le procédé selon l'invention est caractérisé en ce qu'il comprend une étape supplémentaire de broyage de l'extrait et de récupération d'un broyat de cellules présentant une teneur en mimosine inférieure à 5 ng/g de matière sèche.
Dans un mode particulier de réalisation, le procédé selon l'invention est caractérisé en ce que le broyat obtenu est soumis à une séparation liquide/solide suivie de la récupération de la phase liquide comme extrait cellulaire présentant une teneur en mimosine inférieure à 5 ng/g de matière sèche.
Dans un autre mode particulier de réalisation, le procédé selon l'invention est caractérisé en ce que le broyat obtenu est soumis à une séparation liquide/solide suivie de la récupération de la phase solide de débris cellulaires comme extrait cellulaire présentant une teneur en mimosine inférieure à 5 ng/g de matière sèche.
Dans un mode particulier de réalisation, le procédé selon l'invention est caractérisé en ce que le milieu de culture de l'étape c) et/ou de l'étape d) comprend un substrat de la phényl-ammonia-lyase.
Dans un mode particulier de réalisation, le procédé selon l'invention est caractérisé en ce que le substrat de la phényl-ammonia-lyase est choisi dans le groupe constitué par la phénylalanine, en particulier la L-phénylalanine, l'acide cinnamique, l'acide aspartique, l'acide glutamique ainsi que leurs mélanges.
Dans un mode particulier de réalisation, le procédé selon l'invention est caractérisé en ce que l'extrait obtenu contient au moins un acide aminé N-phenylpropenoyl.
Dans un mode particulier de réalisation, le procédé selon l'invention est caractérisé en ce que l'acide aminé N-phénylpropenoyl est choisi dans le groupe constitué par :

P1 : 1-*O*-(4-coumaroyl)-β-D-glucose

$C_{15}H_{18}O_8$

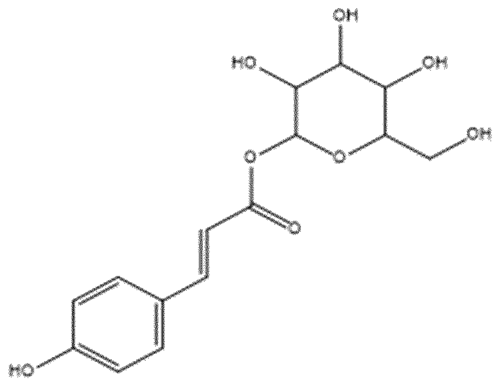

P2 : *N-p*-Coumaroylaspartic acid ou Aspartic acid; (S)-form, N-(4-Hydroxycinnamoyl)
$C_{13}H_{13}NO_6$

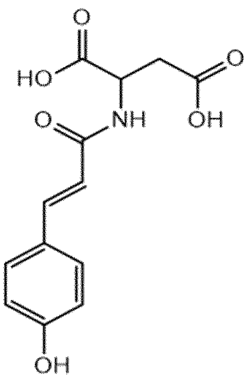

P3 : *N-cis*-(*p*-Coumaroyl)glutamic acid ou Glutamic acid; (*S*)-form, *N*-(4-Hydroxy-Z-cinnamoyl)
$C_{14}H_{15}NO_6$

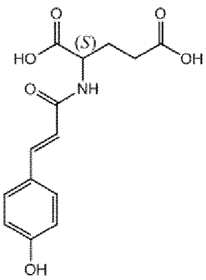

P5 : 4-hydroxycinnamide
$C_9H_9NO_2$

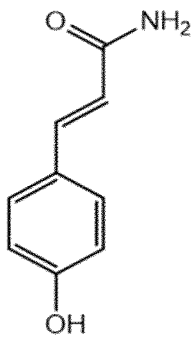

P6 : Glutamic acid; (*S*)-form, *N*-cinnamoyl
$C_{14}H_{14}NO_5$

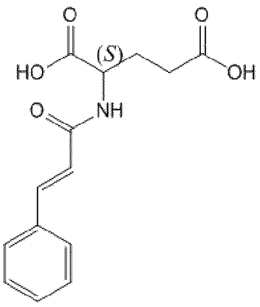

ainsi que leurs mélanges.

C'est aussi un objet de la présente invention que de fournir un extrait de culture in vitro de cellules de Mimosa pudica susceptible d'être obtenu par un procédé selon la présente invention et présentant une teneur en mimosine inférieure à 5 ng/g de matière sèche.

L'invention a aussi pour objet une extrait de culture in vitro de cellules de Mimosa pudica, caractérisé en ce que sa teneur en mimosine est inférieure à 5 ng/g de matière sèche.

Dans un mode de réalisation particulier, l'extrait de culture in vitro de cellules de Mimosa pudica selon l'invention est caractérisé en ce qu'il contient au moins un acide aminé N-phenylpropenoyl.

Dans un mode de réalisation particulier, l'extrait selon l'invention est aussi caractérisé en ce que le au moins un acide aminé N-phenylpropenoyl est choisi dans le groupe constitué par :

P1 : 1-*O*-(4-coumaroyl)-β-D-glucose
$C_{15}H_{18}O_8$

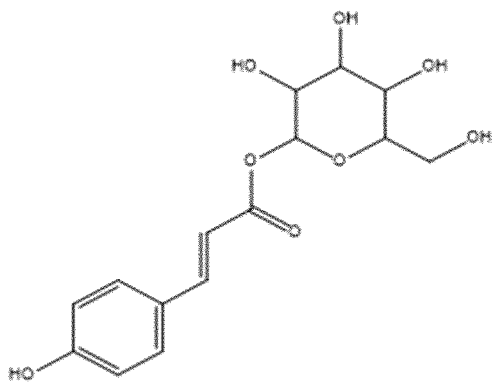

P2 : *N*-*p*-Coumaroylaspartic acid ou Aspartic acid; (S)-form, N-(4-Hydroxycinnamoyl)
$C_{13}H_{13}NO_6$

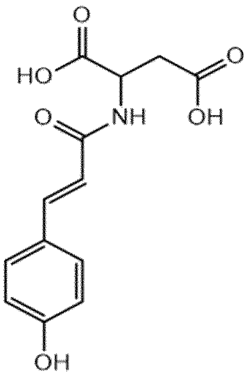

P3 : *N-cis*-(*p*-Coumaroyl)glutamic acid ou Glutamic acid; (S)-form, *N*-(4-Hydroxy-Z-cinnamoyl)
$C_{14}H_{15}NO_6$

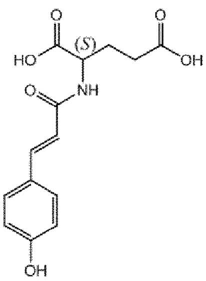

P5 : 4-hydroxycinnamide
$C_9H_9NO_2$

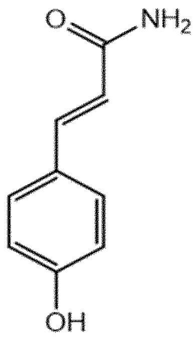

P6 : Glutamic acid; (*S*)-form, *N*-cinnamoyl
$C_{14}H_{14}NO_5$

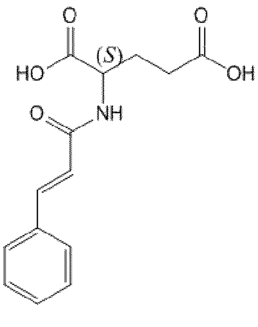

ainsi que leurs mélanges.

Selon un mode particulier de réalisation, l'extrait selon l'invention est caractérisé en ce que la teneur totale de l'extrait en au moins un acide aminé N-phenylpropenoyl est comprise entre 1 et 50 µg/g de biomasse sèche, et plus préférentiellement entre 10 et 40 µg/g de biomasse sèche.

Selon un mode particulier de réalisation, l'extrait selon l'invention est caractérisé en ce que les cellules sont des cellules indifférenciées.

C'est aussi un objet de l'invention que de viser un extrait tel que décrit ici pour son utilisation dans le traitement de troubles inflammatoires de la peau.

C'est aussi un objet de l'invention que de viser un extrait tel que décrit ici pour son utilisation comme inhibiteur de la Thymic Stromal lymphopoietine.

Dans un mode de réalisation particulier, les troubles inflammatoires de la peau sont choisis parmi la dermatite atopique, le prurit, le psoriasis.

Une Composition dermatologique pour le traitement d'un trouble inflammatoire de la peau choisi parmi la dermatite atopique, le prurit, le psoriasis, comprenant un extrait selon l'invention en quantité efficace et au moins un excipient dermatologique est aussi un objet de la présente invention.

La présente invention vise encore l'utilisation non-thérapeutique d'un extrait selon l'invention et tel que décrit ici pour le traitement cosmétique du vieillissement cutané et des troubles cutanés associés à un stress oxydatif de la peau dont le stress oxydatif dû à la pollution environnementale.

La présente invention concerne aussi une composition cosmétique comprenant un extrait selon l'invention associé à un excipient cosmétiquement acceptable. L'invention vise aussi une composition dermatologique comprenant un extrait selon l'invention tel que décrit, associé à un excipient dermatologiquement acceptable.

Enfin, la présente invention concerne encore l'utilisation non-thérapeutique d'une composition selon l'invention pour le traitement cosmétique du vieillissement cutané et des troubles cutanés associés à un stress oxydatif de la peau, tel que le stress oxydatif dû à la pollution environnementales.

Définitions

L'expression « dédifférentiation » s'entend d'un retour des cellules à un état méristématique, c'est-à-dire à l'état de cellules indifférenciées, c'est-à-dire des cellules qui ont perdu leurs caractéristiques morphologiques et qui sont physiologiquement différentes des cellules du tissu originel dont elles faisaient partie.

L'extrait selon l'invention présente une teneur en mimosine inférieure à 5ng/g de matière sèche, plus particulièrement inférieure à 4 ng/g de matière sèche, plus particulièrement encore inférieure à 3 ng/g de matière sèche, encore plus particulièrement encore inférieure à 2 ng/g de matière sèche, voire plus particulièrement encore inférieure à 1 ng/g de matière sèche.

Il est ainsi particulièrement remarquable et étonnant que la mise en culture selon l'invention de cellules de Mimosa pudica à l'état indifférencié permet d'obtenir un extrait quasiment dénué de mimosine et c'est là tout l'intêret de la présente invention du fait de la toxicité de ce composé.

Dans le cadre de la présente invention, l'expression « extrait » vise tout autant le milieu de culture, après avoir cessé la propagation de la culture, principalement constitué des cellules de Mimosa pudica indifférenciées baignant dans le milieu de culture liquide. Un tel extrait contient entre 100 et 500 g de matière sèche par litre, particulièrement entre 150 et 350 g/l. La matière étant principalement constituée de la biomasse de cellules indifférenciées.

L'extrait selon l'invention vise aussi la fraction solide dudit milieu de culture, c'est-à-dire la biomasse constituée des cellules indifférenciées de Mimosa pudica séparées du milieu de culture.

L'extrait selon l'invention vise aussi la fraction liquide dudit milieu de culture, c'est-à-dire le milieu de culture débarrassé des cellules de Mimosa pudica indifférenciées.

Ces fractions solides et liquides peuvent être aisément obtenues par une technique de séparation liquide/solide bien connue de l'homme du métier qui peut être choisie parmi la centrifugation, la décantation, la filtration par exemple.

L'extrait selon l'invention peut aussi est constitué d'un broyat de cellules de Mimosa pudica indifférenciées. Un tel broyat peut être obtenu directement par broyage du milieu de culture après avoir cessé la propagation. Un tel broyat contient donc les débris de membrane et de parois cellulaires, le contenu intracellulaire ainsi que le milieu de culture ainsi que les composés et molécule entrainés.

Un tel broyat constitutif de l'extrait selon un mode particulier de réalisation, peut être obtenu en broyant la fraction solide du milieu de culture, constituée des cellules indifférenciées de Mimosa pudica, telle qu'obtenue ci-avant ainsi que des composés et molécules entrainés.

Un extrait selon l'invention peut encore être constitué par la fraction solide du broyat tel que défini ci-avant. Un tel extrait comprend les fragments de parois et membranes cellulaires ainsi que certains composés et molécules entrainés.

Un extrait selon l'invention peut enfin être représenté par la fraction liquide du broyat après resuspension et broyage de la fraction solide du milieu culture contenant les cellules de Mimosa pudica indifférenciées.

Le broyage peut être effectué par tout moyen connu de l'homme du métier, par mixeur de type Utraturax par exemple, par agitation avec des billes de verres assistée ou non d'ultrasons, par exemple.

Il apparait ainsi que l'extrait selon l'invention peut prendre plusieurs formes.

De manière préférée, l'extrait selon l'invention contient des cellules indifférenciées de Mimosa pudica entières ou des cellules indifférenciées de Mimosa pudica broyées. Cet extrait est caractérisé par son contenu en mimosine inférieur à 5, préférentiellement inférieur à 4, préférentiellement inférieur à 3, plus particulièrement inférieur à 2, plus particulièrement encore inférieur à 1 ng/g de matière sèche.

Dans le cadre d'un extrait selon l'invention constitué de fraction liquide dénuée de fraction solide (cellules entière ou fragments de cellules), la quantité de mimosine est inférieure à 5, préférentiellement inférieur à 4, préférentiellement inférieure à 3, plus particulièrement inférieure à 2, plus particulièrement encore inférieure à 1 ng/ml d'extrait.

D'une manière plus générale, les cultures in vitro de tissus végétaux en suspension permettent de produire des composés organiques actifs directement issus du métabolisme primaire ou secondaire des cellules.

Les cellules végétales en suspension sont maintenues totipotentes dans un état indifférencié similaire à celui des cellules souches pour les cultures de cellules animales. Ces cellules végétales sont donc théoriquement capables de produire tous les métabolites observés dans la plante entière. La dédifférenciation entraîne une perturbation des voies de biosynthèse d'ordre génétique ou épigénétique si bien que les profils chimiques sont différents quantitativement et qualitativement entre la plante entière et les souches cellulaires qui en découlent. Ainsi théoriquement des intermédiaires réactionnels non observés dans la plante entière peuvent apparaître en suspension cellulaire et inversement. Ceci offre une nouvelle richesse et permet d'accéder à une biodiversité phytochimique latente.

Un des aspects de la présente divulgation concerne la préparation issue d'une culture in vitro de cellules indifférenciées de Mimosa pudica.

Par cellules végétales « indifférenciées », ou « dédifférentiées » on entend toute cellule végétale ne présentant aucun caractère d'une spécialisation particulière c'est-à-dire dans un état physiologique proche des tissus méristematiques de la plante à l'état naturel. Ces cellules sont capables de vivre par elles-mêmes et non en dépendance avec d'autres cellules.

La dédifférenciation initiale des cellules de Mimosa pudica est obtenue à partir de matériel végétal vivant prélevé sur la plante ou la jeune pousse, que ce soit la feuille, les pétioles, la tige, la racine, la graine, la fleur et ses organes ou le bourgeon, plus particulièrement à partir de graines ou de feuilles.

Le procédé de culture de cellules dédifférenciées est obtenu in vitro par toute méthode connue par l'homme de l'art, on se référera par exemple à : Murashige, T., Skoog, F. 1962. A revised medium for rapid growth and bio assays with tobacco tissue cultures. Physiol. Plant 15: 473-496. / Plant Culture Media, Vol-1 Formulations and Uses E. F. George, D. J. M. Puttock, and H. J. George (1987) Exegetics Ltd. Edington, Westbury, Wilts, BA134QG England.

Un extrait selon la présente invention peut être obtenu en réalisant les étapes successives suivantes :

a) mise en dédifférentiation des cellules,
b) mise en suspension cellulaire avec un milieu de culture maintenant les cellules dans un état physiologique indifférencié,
c) culture de propagation et production de biomasse avec un milieu de culture
d) dans un mode de réalisation particulier, enrichissement du compartiment intracellulaire de la culture en métabolites d'intérêts par bioconversion
e) obtention de l'extrait.

La préparation peut s'effectuer en erlenmeyer s'il s'agit de produire de faibles quantités de biomasse ou en bioréacteur pour des quantités plus importantes. Par exemple, en erlenmeyer avec 500 ml de suspension cellulaire, on récolte 175 g de biomasse essorée en moyenne (soit 350 g de biomasse /L de suspension cellulaire) alors qu'en bioréacteur de 10 L on récolte 3000 g de biomasse essorée en moyenne (300 g/L de biomasse).

Selon les espèces cultivées et leurs sensibilités aux stress de culture, différents types de bioréacteurs peuvent être utilisés pour améliorer la croissance des tissus et la production de métabolites secondaires. Trois modes principaux sont rencontrés pour la culture de cellules végétales en bioréacteur :

1. la culture discontinue ou batch,

2. la culture recharge/récolte ou fed-batch et

3. la culture continue.

**Etape de stérilisation du matériel végétal :**

Des explants de plantes de Mimosa pudica et plus particulièrement des graines sont prélevées et décontaminées avec des solutions éthanoliques à 70% puis des solutions d'hypochlorite de sodium ou de calcium ou bien des solutions de chlorure de mercure à température ambiante durant plusieurs minutes. Les tissus sont rincés à l'eau distillée stérile puis subissent au moins un lavage à l'eau distillée stérile en fin de décontamination.

**Etape de dédifférentiation des cellules**

Dans le cas d'utilisation de graines, celles-ci sont décontaminées et sont mises à germer avec un milieu gélosé nutritif de Murashige & Skoog, complémenté de saccharose et de facteurs de croissance. Ces derniers vont conditionner la machinerie cellulaire des explants de manière à stimuler les divisions cellulaires et aboutir à des amas cellulaires ou cals dédifférenciés (callogénèse). Les cals obtenus vont être transférés sur un milieu nutritif de dédifférentiation neuf toutes les 3 à 4 semaines. En effet certain constituants de ce milieu gélosés peuvent se trouver métabolisés par les cals ou bien dégradés par l'action de l'air.

Un homme du métier peut aussi utiliser des tissus différentiés tels que des explants issus de feuilles par exemple pour obtenir des cellules indifférentiées.

De façon générale, afin d'obtenir une dédifférenciation rapide et une multiplication cellulaire intense sous la forme de cals friables (callogénèse) qui favorisera un passage en milieu liquide, une composition hormonale à base de facteurs de croissance tels que des auxines (Picloram ou acide 4-Amino-3,5,6-trichloro-2-pyridinecarboxylique) et des cytokinines (kinétine) a été testée avec succès. Les graines stérilisées peuvent être déposées en contact avec le milieu gélosé composé d'un milieu de 30 g/L de saccharose, 8 g/L d'agar, complémenté avec 1,5 mg/L de kinétine et 2 mg/L d'acide 4-Amino-3,5,6-trichloro-2-pyridinecarboxylique (Picloram) et ajusté à pH 6 avant un autoclavage de 20 min à 121°C (1 bar). Les boîtes de pétri contenant les graines sont laissées à incuber à l'obscurité à 28°C. Les premiers cals apparaissent au bout de quelques jours, en particulier 2 semaines. Les cals obtenus sont transférés sur un nouveau milieu toutes les 3 - 4 semaines environ en divisant les cals au scalpel de manière à maintenir une taille d'environ2 à 3 cm. Ces transferts se succèdent pendant environ plusieurs semaines, voire plusieurs mois, par exemple 6 à 8 mois, de manière à obtenir des cals friables.

**Etape de mise en suspension cellulaire dans un milieu de culture**

La dédifférentiation cellulaire au gré des transferts successifs des cals sur milieu gélosé abouti à la formation de cals friables. Cette baisse de la cohésion entre les cellules est une conséquence de la dédifférentiation qui peut survenir entre deux et six mois selon la plante. Cet état est propice au passage en milieu liquide car il garantit un délitement des cals en suspension cellulaire tout en minimisant les stress mécaniques induits. Ainsi une collection de cals friables est introduit (10-20% du volume) dans le milieu nutritif liquide préparé suivant la même formulation que le milieu gélosé de dédifférentiation mais sans agent gélifiant.

Les cals friables sont délités ainsi en milieu liquide par l'action d'une table d'agitation pendant quelques jours et la suspension cellulaire obtenue est débarrassée des parties de cals non délitées formant ainsi une suspension cellulaire homogène. Cette suspension est maintenue en culture de manière à obtenir une population cellulaire suffisamment dense. A ce stade la suspension est (repiquée ou) diluée dans du milieu nutritif neuf et mise en culture de la même manière.

La mise en suspension cellulaire initiale peut être réalisée en déposant environ 20 à 40 g de cals friables dans un erlenmeyer de 500 ml contenant 200 ml de milieu. Les cals friables sont délités ainsi en milieu liquide par l'action d'une table d'agitation pendant 2 à 3 jours à 115 RPM à l'obscurité à 29°C. Ensuite la suspension cellulaire est récoltée à la pipette laissant de côté les amas de cals résiduels non délités. La suspension cellulaire forme ainsi une suspension homogène de micro-amas cellulaires. Cette suspension est maintenue en culture de manière à obtenir une population cellulaire « suffisamment » dense. La suspension cellulaire obtenue est cultivée pendant 14 jours puis propagée par dilution au 1/5ème dans du nouveau milieu sur la même durée. Des ajustements de la composition du milieu de culture (nutriments, facteurs de croissance etc.) ont été réalisés afin de maximiser la productivité en biomasse. Il en résulte le milieu de propagation de la biomasse SENSMS (Cf tableau 1) optimisé pour la suspension cellulaire liquide. Ce milieu est une version modifiée du milieu de Murashige & Skoog pour la callogénèse. Ce milieu est ajusté à pH 6 par l'ajout de KOH suivi d'un autoclavage de 20 min à 121°C (p = 1 bar) ou d'une filtration stérilisante à 0,2$\mu$m.

**Tableau 1** : Milieu SENSMS qui est une version modifiée du milieu de Murashige & Skoog (MS) servant pour la culture en conditions optimales des cellules de sensitive en suspension en erlenmeyer ou en bioréacteur

| **Milieu SENSMS - optimisé pour la croissance cellulaire** | | | |
|---|---|---|---|
| $NH_4NO_3$ | 1,65 | g/L | Macro éléments |
| $KNO_3$ | 1,9 | g/L | |
| $CaCl_2.2H_2O$ | 0,44 | g/L | |
| $MgSO_4.7H_2O$ | 0,37 | g/L | |
| $KH_2PO_4$ | 0,17 | g/L | |
| KI | 0,83 | mg/L | Micro éléments |
| $H_3BO_3$ | 6,2 | mg/L | |
| $MnSO_4.4H_2O$ | 22,3 | mg/L | |
| $ZnSO_4.1\ H_2O$ | 6,61 | mg/L | |
| $Na_2MoO_4.2H_2O$ | 0,25 | mg/L | |
| $CuSO_4.5H_2O$ | 0,025 | mg/L | |
| $CoCl_2.6H_2O$ | 0,025 | mg/L | |
| $FeSO_4.7H_2O$ | 41,7 | mg/L | |
| $Na2EDTA.2H_2O$ | 55,95 | mg/L | |
| myo-Inositol | 150 | mg/L | Vitamines |
| Acide nicotinique | 0,75 | mg/L | |
| Pyridoxine-HCl | 0,75 | mg/L | |
| Thiamine-HCl | 0,75 | mg/L | |
| Glycine | 2 | mg/L | |
| Picloram | 2 | mg/L | Facteurs (hormones de croissance) |
| Kinétine | 1,5 | mg/L | |
| Saccharose | 30 | g/L | Source carbonée |

**Culture de propagation et production de biomasse avec un milieu de culture**

Après plusieurs repiquages de la sorte, la suspension cellulaire est stabilisée lorsque la densité de cellules obtenue sur la période est constante. Des ajustements de la composition du milieu de culture (nutriments, facteurs de croissance...) est alors possible afin de maximiser la productivité en biomasse. Ce milieu optimisé est employé comme moyen de production de biomasse afin d'en extraire les principes actifs.

La culture cellulaire en condition « optimale » ainsi établie est stabilisée et entretenue en erlenmeyer (culture de propagation) à raison d'une dilution de la suspension cellulaire au $1/5^e$ tous les 14 jours. Ceci équivaut à une culture cellulaire inoculée à 60 g/L env. de biomasse fraîche qui produit une suspension cellulaire de 350 g/L env. au bout de 14 jours de culture; ou inoculée en bioréacteur selon les besoins.

La biomasse fraîche représente 100 à 500g par litre de suspension, et de façon plus préférentielle entre 200 et 350 g par litre de suspension.

**Etape optionnelle : Enrichissement du compartiment intracellulaire de la culture en métabolites d'intérêts par bioconversion**

Il est parfois indispensable de fournir aux cellules des « précurseurs » dont la structure est suffisamment proche du produit final dans l'espoir de les voir incorporer, modifier ou transformer à l'aide des équipements enzymatiques présents dans la cellule.

Dans un mode particulier de réalisation, le procédé selon l'invention est caractérisé en ce que le milieu de culture de l'étape c) et/ou de l'étape d) comprend un substrat de la phényl-ammonia-lyase.

Dans un mode particulier de réalisation, le procédé selon l'invention est caractérisé en ce que le substrat de la phényl-ammonia-lyase est choisi dans le groupe constitué par la phénylalanine, en particulier la L-phénylalanine, l'acide cinnamique, l'acide aspartique, l'acide glutamique ainsi que leurs mélanges.

Dans un mode particulier de réalisation, le procédé selon l'invention est caractérisé en ce que l'extrait obtenu contient au moins un acide aminé N-phenylpropenoyl.

Ce procédé de modification d'un précurseur par la cellule porte le nom de bioconversion. L'ajout de précurseurs dans le milieu de culture pourrait être une approche intéressante pour augmenter la production des métabolites secondaires d'intérêts. Ce concept se base sur le principe que n'importe quel composé, qui est un intermédiaire réactionnel dans la voie de biosynthèse du métabolite d'intérêt, peut probablement améliorer le rendement du produit final.

Ainsi, d'après la structure des NPA (voir Figure ci-dessous), certaines molécules (acide-aminé) semblent être des précurseurs de ces intermédiaires réactionnels. Par exemple, l'acide aminé L-phénylalanine représente un précurseur intéressant pour moduler positivement l'activité d'une l'enzyme Phényl-Ammonia-Lyase (PAL). De la même manière, l'acide cinnamique pourrait être à l'origine de ce même phénomène. On peut également envisager multiples combinaisons, notamment avec l'acide aspartique ou l'acide glutamique. Nous avons effectué des nombreuses expériences, utilisant ces différents précurseurs (seuls ou en combinaison), et de manière surprenante avec la L-Phénylalanine, nous obtenons des NPAs intéressants qui n'ont jamais été décrits dans les cellules de Mimosa pudica tels que :

P1 : 1-*O*-(4-coumaroyl)-β-D-glucose
$C_{15}H_{18}O_8$

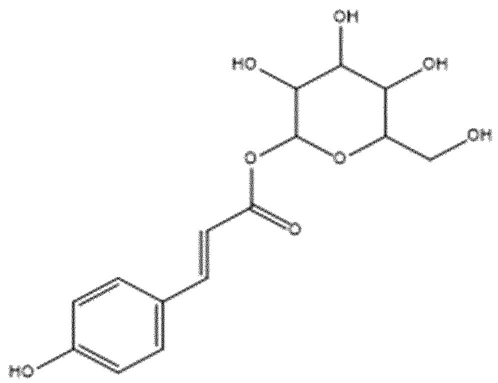

P2 : *N-p*-Coumaroylaspartic acid ou Aspartic acid; (S)-form, N-(4-Hydroxycinnamoyl)
$C_{13}H_{13}NO_6$

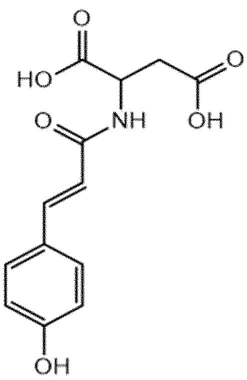

P3 : *N-cis*-(*p*-Coumaroyl)glutamic acid ou Glutamic acid; (*S*)-form, *N*-(4-Hydroxy-Z-cinnamoyl)
$C_{14}H_{15}NO_6$

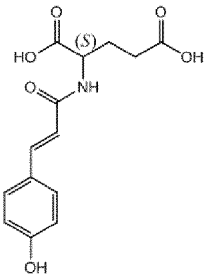

P5 : 4-hydroxycinnamide
$C_9H_9NO_2$

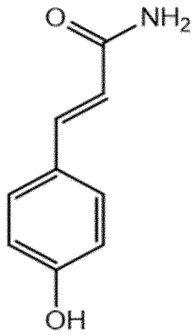

P6 : Glutamic acid; (*S*)-form, *N*-cinnamoyl
$C_{14}H_{14}NO_5$

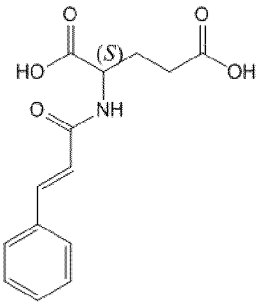

ainsi que leurs mélanges.

**Obtention de l'extrait**

Après arrêt de la propagation, la suspension cellulaire obtenue peut être filtrée ou centrifugée afin de la séparer du milieu de culture et obtenir d'une part le milieu extracellulaire (ou surnageant de culture) et d'autre part la biomasse récoltée. La biomasse peut alors être traitée de différentes manières selon l'utilisation. Soit elle est séchée par lyophilisation, soit elle est remise en suspension, par exemple à 30% dans de la Glycérine 68.2% avec 0.8 % de carraghénane et 1% d'acide citrique. On peut aussi réaliser une extraction par solvant sur la suspension cellulaire en faisant au préalable un broyage cellulaire par sonication ou par French press par exemple.

**Formulation**

Un autre objet de l'invention concerne une composition cosmétique ou dermatologique comprenant une extrait de culture in vitro de cellules de Mimosa pudica susceptible d'être obtenu par le procédé selon l'invention et un ou plusieurs excipients cosmétiquement et/ou dermatologiquement acceptables, et de préférence destinés à une application topique.

Les excipients cosmétiquement et/ou dermatologiquement acceptables peuvent être tout excipient parmi ceux connus de l'homme de l'art. La composition selon l'invention sera notamment une composition topique notamment sous

forme de crème, d'une lotion, d'un gel, d'une pommade, d'une émulsion, d'une microémulsion, d'un spray, etc.

La composition cosmétique ou dermatologique selon l'invention peut en particulier contenir des additifs et aides à la formulation, tels que des émulsionnants, des épaississants, des gélifiants, des fixateurs d'eau, des agents d'étalement, des stabilisants, des colorants, des parfums et des conservateurs.

Un autre objet selon l'invention est l'utilisation d'un extrait selon l'invention dans le traitement des troubles inflammatoires cutanés, comme agent anti-oxydant dont le traitement des stress oxydatifs dus à la pollution environnementale, et comme agent anti-âge.

Par « trouble inflammatoire cutané », on entend la dermatite atopique, prurit, et démangeaisons dues au prurit (inhibition TSLP), l'eczéma et le psoriasis.

De manière préférée, lesdits troubles inflammatoires dermatologiques consistent en la dermatite atopique, le prurit, l'eczéma ou le psoriasis. Selon un autre mode de réalisation, l'invention objet de la présente demande de brevet vise une composition comprenant au moins, à titre de principe actif, un extrait selon l'invention.

L'invention concerne donc, de manière préférée, une composition cosmétique ou dermatologique. La composition selon l'invention vise le traitement de troubles inflammatoires dermatologiques.

De manière préférée, lesdits troubles inflammatoires dermatologiques consistent en la dermatite atopique, le prurit, les démangeaisons dues au prurit, l'eczéma ou le psoriasis.

La composition selon l'invention peut être préparée sous la forme d'une émulsion eau dans huile (E/H) ou huile dans eau (H/E), d'une émulsion multiple comme par exemple, une émulsion eau dans huile dans eau (E/H/E) ou une émulsion huile dans eau dans huile (H/E/H), d'une micro-émulsion ou encore sous la forme d'une hydrodispersion ou une lipodispersion, un gel ou un aérosol. Les excipients dermatologiquement ou cosmétiquement compatibles peuvent être tout excipient parmi ceux connus de l'homme de l'art en vue d'obtenir une composition pour l'application topique sous forme d'un lait, d'une crème, d'un baume, d'une huile, d'une lotion, d'un gel, d'un gel moussant, d'une pommade, d'un spray, etc.

Outre des compositions dermatologiques et cosmétiques, l'invention vise également des compositions pharmaceutiques pour une utilisation à titre de médicament. L'invention vise donc une composition pharmaceutique comprenant, en outre, un véhicule pharmaceutiquement acceptable. Dans la présente description, on entend désigner par véhicule pharmaceutiquement acceptable, un composé ou une combinaison de composés entrant dans une composition pharmaceutique ne provoquant pas de réactions secondaires et qui permet par exemple la facilitation de l'administration du ou des composés actifs, l'augmentation de sa durée de vie et/ou de son efficacité dans l'organisme, l'augmentation de sa solubilité en solution ou encore l'amélioration de sa conservation. Ces véhicules pharmaceutiquement acceptables sont bien connus et seront adaptés par l'homme de l'art en fonction de la nature et du mode d'administration.

**EXEMPLE : Culture de cellules végétales en bioréacteur WAVE (5 L)**

Matériels et méthodes : Le réacteur WAVE (Sartorius) de volume utile 5 L contenant 4 L de milieu MS SENS (composition voir tableau plus haut) a été inoculé par 1 L de suspension de culture de cellules de M pudica (densité cellulaire entre 300 et 400 g/L en Fresh weight FW)

Paramètres :

- Température : 27°C
- Volume d'air : 0.5 L/mn (lpm)
- Pression : 5 mPa à 10 mPa
- Angle d'agitation: 7°
- $pO_2$ maintenue à 75% par augmentation des rpm entre J0 (19 rpm) et J9 (27 rpm)
- $pO_2$ maintenue à 75% par enrichissement de l'air en $O_2$ entre J9 (5% $O_2$) et J15 (20% $O_2$) à 25 rpm

En suivant la cinétique de croissance on observe l'augmentation de la biomasse évaluée par « fresh weight » ou « dry weight » (FW ou DW) corrélée avec la consommation en sucre (décroissance de la courbe) jusqu'au 13$^{ème}$ jour de culture, J13, où le milieu de bioconversion est ajouté.

**Bioconversion et récolte**

Après 13 jours (J13) de culture en batch d'une culture de 4 L, la concentration cellulaire se trouve entre 300 et 350 g/L (FW) équivalent à 12 à 14 g/L (DW). Lorsque cette concentration cellulaire est atteinte, on procède à la bioconversion en injectant les précurseurs par filtration stérilisante, nous avons sélectionné les différents acides aminés (acide aspartique, acide cinnamique, acide glutamique ou L-Phénylalanine), et dans nos conditions de culture, la L-Phénylalanine a donné les meilleurs résultats en terme de rendement en bioconversion en NPAs. Ci-après un exemple de concentrations en précurseurs suivantes :

| Préparation extemporanée | Concentration(SM) | Concentration (Suspension) | Volume final de SM (**ml**) |
|---|---|---|---|
| **$NH_4H_2PO_4$** | 50 g/L | 0,1 g/L | 10 |
| **L-Phénylalanine** | 300 mM | 6 mM | 100 |
| **Saccharose** | 500 g/L | 10 g/L | 100 |

**Milieu de Bioconversion favorisant les acides hydroxycinnamiques dont leurs dérivés, les N-Phénylpropenoyl Aminoacids (NPA)**

Diluer les composés dans H2O et ajouter par filtration stérilisante le mix dans le bioréacteur. Après 48h, c'est-à-dire entre J15 et J16, procéder à la récolte de la biomasse par filtration directe à l'aide d'une « poche en Nylon 20 μm » par exemple. La biomasse est lavée 1 fois avec un volume d'eau déminéralisée stérile équivalent au volume de biomasse récoltée. La concentration cellulaire évaluée à J13 doit être équivalente (+/-10%) à J15. Tout au long de la culture, la baisse du volume de suspension est principallement due aux prélèvements effectués.

On prélève un volume de la culture à J13, J14, J15 et J16, on extrait ensuite avec un solvant puis les différents extraits ont été analysés par HPLC couplé avec la spectrométrie de masse. On voit dans les 2 graphes ci-dessous que par rapport à J13 (le jour de l'ajout du précurseur L-Phénylalanine), l'apparition progressive des nouveaux pics J14, J15 jusqu'à atteindre le maximum d'intensité au jour J16.

Les pics P1, P2, P3, P4, P5 et P6 ont été isolés à J16, analysés par spectrométrie de masse et par RMN afin de déterminer la structure de la molécules. Le Pic P4 étant en très faible quantité celui ci ne nous a pas permis de déterminer sa structure. Pour la 1[e] fois et de manière surprenante, nous découvrons que le précurseur L-Phénylalanine était le meilleur acide-aminé pour la bioconversion.

Les produits NPAs P1, P2, P3, P5 et P6 ont été identifiés par spectroscopie de masse et RMN.

Liste des noms des NPAs :

P1 : 1-*O*-(4-coumaroyl)-β-D-glucose
$C_{15}H_{18}O_8$

P2 : *N*-*p*-Coumaroylaspartic acid ou Aspartic acid; (*S*)-form, *N*-(4-Hydroxycinnamoyl)
$C_{13}H_{13}NO_6$

P3 : *N*-*cis*-(*p*-Coumaroyl)glutamic acid ou Glutamic acid; (*S*)-form, *N*-(4-Hydroxy-*Z*-cinnamoyl)
$C_{14}H_{15}NO_6$

P5 : 4-hydroxycinnamide
$C_9H_9NO_2$

P6 :

Glutamic acid; (*S*)-form, *N*-cinnamoyl
C14H14NO5

**EXEMPLE 2: DOSAGES DE MIMOSINE DE CELLULES VEGETALES VERSUS FEUILLES DE MIMOSA PUDICA**

L'objectif est de mettre au point les conditions analytiques qui permettent d'identifier et de quantifier la mimosine dans les extraits. La biomasse issue de culture en suspension de cellules de M pudica (avec bioconversion) a été extraite avec ETOH80 ou avec ETOH60. Les feuilles séchées de M pudica ont été séchées, broyées et extraites avec les mêmes solvants. On se sert de référence la L-Mimosine de Sigma pour l'identification et quantification. Les échantillons ont été analysés par HPLC/spectrométrie de masse.

Matériels et méthodes : Conditions Analytiques

Spectromètre de masse TripleTOF 4600 ABSciex
Méthode (ES+): TOF (100ms) / MRM (50ms)
Colonne: Acquity HSS C18, 1.8μm, 2.1x100mm, fritté 0.5μm

Eluants : A ($H_2O$-0.1%$HCO_2H$) / B ($CH_3CN$-0.1%$HCO_2H$)
Débit : 500 μl/min
Injections : 10 μl
Gradient :

| Time (mn) | %A | %B |
|---|---|---|
| 0.0 | 100 | 0 |
| 1.0 | 100 | 0 |
| 4.0 | 10 | 90 |
| 4.01 | 0 | 100 |
| 5.0 | 0 | 100 |

Les dosages quantitatifs ont permis de déterminer la limite basse de quantification (LLOQ lower limit of quantification) atteinte dans la solution témoin est de 5 ng/mL. Cette LLOQ et le mode de préparation de nos échantillons hydro-éthanolique permettent de confirmer l'absence de Mimosine dans nos échantillons de culture cellulaire à la limite de détection (1 ng de Mimosine par g de biomasse) :

- Extraits EtOH80 et EtOH60 de cellules (20% w/w) <1 ng/g de cellules fraîches
- Extrait EtOH60 de cellules lyophilisées (5% w/w) <1 ng/g de cellules lyophilisées
- Extrait EtOH60 de plantes = 2160 ng/g de plante sèche.
- Extrait EtOH80 de plantes = 780 ng/g de plante sèche

Les analyses ont été effectués de la même manière sur les CCV sans bioconversion (ie sans ajout de précuseurs de NPA) et ont démontré que la teneur en mimosine est également < 1ng/g de cellules fraîches ou lyophilisées.
En conclusion, dans nos cultures de cellules végétales de M pudica, de manière surprenante nous n'avons pas détecté d'élément indésirable comme la Mimosine.

**Exemple 2** - **Activités anti-inflammatoires** : **comparaison extrait plantes vs extrait CCV**

Dans cet exemple, nous comparons l'activité anti-inflammatoire de 2 extraits de *Mimosa* Pudica (E11 et E13) . L'extrait E11 provient de l'extraction à l'Acétate d'Ethyle des feuilles séchées et broyées de Mimosa pudica et l'extrait E13 provient de l'extraction avec le même solvant des broyats issus de culture de cellules végétales de Mimosa pudica sans bioconversion en erlenmeyer. Les 2 extraits ont été pesés après évaporation du solvant à sec. Ils ont été repris dans du DMSO. Pour évaluer et comparer l'activité anti-inflammatoire des 2 extraits dans les mêmes concentrations, nous avons un test pharmacologique in vitro qui consiste à stimuler les macrophages murins, les cellules RAW264.7 qui expriment à leur surface le récepteur TLR4 par le LPS bactérien selon (Kang et al. 2002. J Pharmacol Exp Ther. 302:138-144). Plusieurs paramètres ont été étudiés et comparés à un anti-inflammatoire de référence, la dexaméthasone :

- la production de nitrites par la technique de Griess. Le taux de nitrites reflète le niveau de synthèse de NO induit par la nitrite oxide synthase inductible (iNOS).
- la sécrétion de cytokines: l'interleukine-6 par un dosage ELISA
- la production de TNF-alpha par dosage Multiplex

**CULTURE CELLULAIRE**

Les cellules RAW264.7 sont des macrophages murins en lignée. Ces cellules sont adhérentes et cultivées en plaque 12 puits à 100000 cellules/$cm^2$ (comptage au scepter de Millipore) dans du milieu DMEM supplémenté avec 10% de SVF, de la L. glutamine à 2mM et de la gentamicine à 50 μg/mL.
A environ 70% de confluence, les cellules sont traitées par les extraits de *Mimosa Pudica* E11 (plante) ou E13(CCV) dans les mêmes concentrations (en poids sec) ou avec l'anti-inflammatoire de référence la déxaméthasone à 1 μM (Biovison 1042-1) 1 h avant d'être activées par du LPS de [*E.coli* 055:B5] à 0.2 μg/mL. Les cellules sont incubées à 37°C sous 5% de CO2. Au bout de 24 h, les surnageants cellulaires sont récupérés dans la glace, centrifugés pendant 5 min à 3000 rpm à 4°C, aliquotés puis stockés à -80°C. Les essais cellulaires ont été reproduits 3 fois, dont deux fois en duplicats. Les valeurs moyennes des résultats sont présentées dans les graphes.

La viabilité cellulaire est contrôlée par un test métabolique au MTT (3-[4,5-diméthylthiazol-2yl]-2,5-diphényltétrazolium bromide) avec l'aide du *kit SIGMA CGD1* (kit basé sur l'activité d'une enzyme, la succinate déshydrogénase mitochondriale). Ce test a été fait au préalable afin de déterminer les doses des extraits à tester dans ce modèle.

## DOSAGE DES NITRITES

Le niveau de synthèse de NO est évalué dans des surnageants cellulaires frais ou congelés à -80°C, sans incidence sur le dosage. Le principe du dosage est basé sur la réaction de Griess (réaction de diazotation) qui aboutit à la formation d'un composé azoïque de couleur rose absorbant à 540 nm.

## DOSAGE DE L'IL6

L'IL-6 est dosée dans les surnageants cellulaires dilués au 1/200$^{ième}$ par ELISA colorimétrique, suivant le protocole du fournisseur (R&D Systems, kit M6000B).

## DOSAGE TNF ALPHA

Le dosage simultané dans les surnageants cellulaires du TNF-alpha a été effectué grâce à la technologie xMAP (Multi-Analyte Profiling) de Luminex qui repose sur les principes de la cytométrie en flux et du dosage ELISA en microplaque à 96 puits. Les microbilles utilisées comme support incorporent deux fluorochromes selon un ratio précis, ce qui leur confère un code couleur les identifiant (fluorescences différentes). Le système optique du cytomètre (Bio-Plex 200) est constitué de deux lasers : un laser rouge ($\lambda$ = 635 nm) excite dans chaque microbille le mélange de colorants qui la définie, et identifie ainsi la cytokine à doser. Le second laser, vert, ($\lambda$ = 532 nm) excite le fluorochrome rapporteur attaché à l'anticorps spécifique de détection afin de quantifier la cytokine. Le système est piloté par un ordinateur muni d'un logiciel d'acquisition et d'analyse des données (Bio-Plex Manager version 4.1).

Après décongélation, les surnageants ont été testés dilués au 1/10$^{ème}$ et au 1/40$^{ème}$ en milieu de culture, grâce à un kit Milliplex (MILLIPORE, référence MCYTOMAG-70K-04). Celui-ci comprend les billes spécifiques, les anticorps de détection et les standards pour doser les cytokines.

## RESULTATS

Les concentrations sont exprimées en moyennes. Les pourcentages d'inhibition, lorsque cités pour les extraits, sont rapportés au témoin DMSO 0.2%. Le DMSO est utilisé pour dissoudre l'échantillon sec au préalable avant dilution dans tampon aqueux pour les tests cellulaires.

### Légendes sur les figures :

FIGURE 1 : DOSAGE DES NITRITES

FIGURE 2 : DOSAGE DE L'IL6

FIGURE 3 : DOSAGE DE TNF-alpha

FIGURE 4 : Activité antioxydante

Axes des abscisses des différents échantillons (de gauche à droite) :

- **O** : contrôle négatif - culture de cellules RAW246.7 activées par du LPS à 0.2 $\mu$g/mL.
- **DEXA :** contrôle positif - incubation des cellules avec la Déxaméthasone à 1 $\mu$M (Biovison 1042-1) 1 h avant d'être activées par du LPS à 0.2 $\mu$g/mL.
- **DMSO :** incubation avec le contrôle solvant servant à solubiliser (E11 et E12) 1 h avant d'être activées par du LPS à 0.2 $\mu$g/mL.
- **E11** : incubation avec extrait issus de feuilles de M pudica (**50 $\mu$g/mL**) 1 h avant d'être activées par du LPS à 0.2 $\mu$g/mL.
- **E13 :** avec extrait issus de cellules végétales (**50 $\mu$g/mL**) 1 h avant d'être activées par du LPS à 0.2 $\mu$g/mL.

**DOSAGE DES NITRITES**

La production de NO est exclusivement inductible et inhibable de façon significative par la déxaméthasone (%i = 35). Le DMSO 0.2% n'a pas d'impact sur le dosage. L'extrait de la plante E11 inhibe faiblement (%i = 16%) la production de nitrites alors que l'extraitCCV E13 inhibe la production de nitrites de façon équivalente et proche de l'inhibition obtenue avec l'anti-inflammatoire de référence (%i = 32%).

**DOSAGE ELISA DE L'IL-6**

Les cellules activées (O) avec du LPS sécrètent d'IL6. La déxaméthasone (DEXA) inhibe la sécrétion d'IL-6 (%i = 38%). Les extraits inhibent la sécrétion d'IL-6 pour E11 (%i = 24%) et pour E13 (%i = 31%)

**DOSAGE LUMINEX de TNF-alpha**

Les RAW264.7 sécrètent du TNF-alpha à l'état basal (0.23 ng/mL de moyenne). Cette production est activée par le LPS 0.2 μg/mL jusqu'à 10 ng/ml en TNF-alpha. Cette activation est faiblement inhibable par la déxaméthasone (%i = 16%). L'extrait CCV E13 diminue la production de TNF-alpha (%i = 40%). A l'inverse l'extrait de la plante E11 augmente la production de TNF-alpha et semble potentialiser l'activité du LPS (+112%).

**En conclusion,** les résultats démontrent que les 2 extraits E11 et E13 à la même concentration inhibent fortement l'inflammation qui se traduisent dans ce modèle par : l'inhibition des nitrites NO, inhibition de la cytokine pro-inflammatoire IL6 comme le contrôle DEXA. De manière surprenante, dans ces conditions expérimentales, l'extrait CCV E13 mieux que le DEXA, inhibe le TNF- alpha alors que l'extrait de la plante E11 le potentialise.

**Exemple 3 : activité anti-oxydante - test ORAC**

L'activité anti-oxydante a été évaluée selon un test ORAC (Oxygen Radical Absorbance Capacity) (Dévalos A. et Al; Polish journal of food and nutrition sciences; 2003;12/53 :133-136). L'indice ORAC permet d'évaluer la capacité antioxydante d'un extrait. Le AAPH (2,2'-azobis-2-methyl-propanimidamide, dihydrochloride) est à l'origine de radicaux libre peroxyl. Dans ce test il est utilisé pour mimer la cinétique de dégradation de la fluorescéine par les radicaux libres. Ceci se traduit par une diminution de fluorescence en fonction du temps et la concentration de l'extrait ou de la référence testée (ici Trolox). Le Trolox (analogue de la vitamine E) est connu comme un anti-oxydant fort. L'ajout de Trolox (gamme de référence) ou d'extrait protège la fluorescéine de la dégradation. Ceci permet d'évaluer une activité anti oxydante mesurée par rapport à une gamme de Trolox.

- Extraits de CCV de Mimosa pudica avec étape de bioconversion (les NPA). Lot WO2. Prélèvements à J13, jour de la bioconversion, à J14 à 10h00, à J14 à 16h00, J15, 16 et J17.
- Dilution des extraits prélevés dans l'eau.

**Test ORAC**

Solutions Utilisées : toutes les solutions sont réalisées dans du tampon phosphate 75 mM pH 7.6. Fluorescéine à 1.17 μM (Utilisation d'une solution mère à 117 mM conservation 1 semaine à 4°C). AAPH à 125 mM à préparer extemporanément. Trolox à 1 mM, (conservation -20°C)

Préparation de la gamme de Trolox :

| Tubes | Trolox 1 mM (μ1) | Tampon phosphate (μl) | [ ] en Trolox μM | pmoles de Trolox |
|---|---|---|---|---|
| 1 | 40 | 960 | 40 | 800 |
| 2 | 30 | 970 | 30 | 600 |
| 3 | 20 | 980 | 20 | 400 |
| 4 | 10 | 990 | 10 | 200 |

| | | | | |
|---|---|---|---|---|
| 5 | 5 | 995 | 5 | 100 |
| 6 | 2,5 | 997,5 | 2,5 | 50 |
| 7 | 0 | 1000 | 0 | 0 |

Volume réactionnel : 200 µl

Dans une plaque 96 puits noir on dépose: 20 µl d'anti-oxydant (gamme de Trolox ou extrait à tester (milieu de culture de cellules broyées)) + 160 µl de fluorescéine à 1,17 µM. Incuber la plaque recouverte d'un film 15 mn à 37°C. Ajouter 20µl / puits de AAPH à 125 mM. Incuber immédiatement à 37°C dans un spectrofluorimètre (SpectraMax) faire une lecture toutes les minutes pendant 90 mn à une longueur d'onde d'excitation de 485 nm et une longueur d'onde d'émission à 520 nm. Chaque essai est réalisé en triplicata. Calculer les aires sous la courbe (AUC) pour chacun des essais (gamme de Trolox ou échantillons). Déterminer les AUC nets = AUC point de gamme ou échantillon - AUC blanc. Tracer la droite d'étalonnage : concentration en Trolox (µM) en fonction de l'AUC net

L'équation de la droite est utilisée pour déterminer un équivalent en Trolox (µM) pour chacun des échantillons dosés.

$$\text{Eq Trolox en µM} = a \times (\text{Auc net})\ 2 + b \times (\text{AUC net})$$

(a et b dont déterminés par l'équation de la droite).

L'indice ORAC correspond à des µmoles de Trolox / 100g d'extrait

**Indice ORAC (TEAC)** = eq Trolox en µM X 20 X (100 000 / [de l'extrait testé] X 20)

La FIGURE 4 montre l'activité anti-oxydante d'extrait contant 75 mg de matière sèche par ml, dilués à 1/200eme ; avant et après étape de bioconversion.

**Résultats** : en abscisse un extrait CCV WO2 prélevé après X jour de culture (heure) J13, J14 à 10h00, J14 à 16h00, et à chaque 24 h après J15, J16, J17 et l'activité du dernier échantillon le control blanc ETOH 40% seul (pas d'activité anti-oxydante). On constate que à J14 après 13 jours de culture et 1 jour après la bioconversion (ajout d'AA), le TEAC (éq µM de TROLOX) a **quintuplé** pour se stabiliser jusqu'à J16-J17. En conclusion, la bioconversion a permis de potentialiser l'activité anti-oxydante grâce à la présence des NPA.

**EXEMPLE 4 : Pharmacologie - Activité modèle Dermatite atopique (DA) in vitro induit**

Evaluation multiparamétrique de l'activité anti-inflammatoire de Mimosa pudica sur un modèle in vitro présentant un phénotype type dermatite atopique. Le modèle pharmacologique a été décrit par Castex-Rizzi et col. (Br J Dermatol. 2014. 170 Suppl 1:12-8)

4.1. Les extraits et composés

Les extraits CCV prélevés de milieu de culture, broyé, séché, étalonné de Mimosa pudica **W01J15** (avec bioconversion) ont été dissous/dilués en solution dans de l'EtOH 40% aux concentrations initiales de 75 mg/ml. Les composés ont été solubilisés extemporanément pour les tests de viabillité ainsi qu'à la dose adéquate pour mesurer l'action pharmacologique sur le modèle dermatite atopique induit.

4.2. Type cellulaire

Kératinocytes épidermiques humains normaux (NHEK) de Lonza. Les cellules sont amplifiées dans des conditions standard de culture.

4.3. Induction d'un phénotype Dermatite Atopique

Les cellules NHEK sont ensemencées et cultivées en milieu de culture Kératinocyte-SFM. Le milieu de culture est ensuite remplacé par du milieu contenant les composés et extraits à tester ou solvant utilisé en contrôle (EtOH40%

en concentration équivalentes à celles utilisées lors du traitement par les composés). Après une "pré-incubation" de 1h le mélange d'inducteur de l'inflammation (Poly (I:C), IL4, IL13) est ajouté et les cellules sont cultivées pendant 24h.

Un contrôle sans inducteur et sans composé est également réalisé en parallèle, nous permettant de valider le modèle induit (NHEK vs NHEK + inducteurs).

Des cellules NHEKs sont également traitées avec un produit de référence, la déxaméthasone à 0.29 mM, et utilisées en contrôle d'efficacité.

L'ARN est extrait des cellules après une incubation de 24 heures avec le mélange d'inducteur.

4.5 Analyse de l'expression différentielle par RT-qPCR

4.5.1. Extraction des ARNs totaux et synthèse des ADNc

L'extraction a été réalisée à l'aide du RNABle® (Eurobio) et du kit RNeasy Mini Kit de QIAGEN. Les ARN totaux extraits sont dosés sur spectrophotomètre (NanoDrop, ND1000, Thermo Scientific) et leurs qualités analysées sur gel d'agarose.

4.5.2. La technique de PCR quantitative

• Principe

La PCR en temps réel est une méthode précise, sensible et rapide qui permet la quantification relative du taux d'expression d'un gène cible par rapport à celui d'un gène de référence exprimé de façon ubiquitaire. Cette technique permet de quantifier l'ARN messager. Cette opération s'effectue après transcription inverse des ARNs en ADN complémentaires par extension de deux amorces situées de part et d'autre de la cible à amplifier grâce à une ADN polymérase. L'incorporation d'un fluorophore (SYBR Green), lors de l'étape d'hybridation de l'amplification exponentielle, permet la quantification et le suivi en temps réel de la quantité de produit d'amplification néoformé. Les valeurs quantitatives sont obtenues à partir du nombre de cycles seuil (Ct : pour Cycle threshold) auquel l'augmentation du signal, associée à une croissance exponentielle du produit de PCR, commence à être détectée en utilisant un programme d'analyse Biosystems PE selon le manuel du fabriquant. Ainsi, plus la quantité d'ADNc du gène cible à l'instant zéro est importante, plus le nombre de Ct est faible (nombre de cycle pour atteindre le seuil).

Afin de standardiser les analyses effectuées en RT-PCR quantitative, il est nécessaire de quantifier dans la même expérience au moins un contrôle endogène appelé "gène de référence". Ce "gène de référence" doit avoir une expression constitutive indépendante de la situation traitée ou non traitée des cellules. L'expression relative des gènes cibles sélectionnés est calculée par la méthode ΔΔCt avec l'aide du logiciel RQ (pour Relative Quantification) fournie par le fabricant (Applied Biosystems). Les valeurs d'expression de chaque gène induites par un composé donné sont également normalisées de telle manière que la valeur des cellules NHEK contrôles (cellules non traitées et cellules traitées avec du DMSO en % identique à celui servant de véhicule au composé) soit égale à 1. Ainsi, la valeur RQ obtenue pour chaque composé pour un gène donné, représente l'expression relative de ce gène après traitement par rapport aux cellules contrôles dont l'expression est 1.

• Choix des amorces

Le choix des amorces a été réalisé avec l'assistance de programmes informatiques dont Oligo 4 (National Biosciences, Plymouth, MN). Les critères de sélection concernent la taille du fragment à amplifier (entre 80 et 120 nucléotides), la taille des amorces (entre 21 et 25 nucléotides), la température d'hybridation des amorces (environ 65°C) et la position des amorces ; en effet, les amorces sont dessinées de façon à ce que l'une des 2 amorces soit à cheval sur un intron et un exon ou que les 2 amorces soient dans deux exons différents si l'intron les séparant est supérieur à 2 Kbp. Le choix de la position bien spécifique des amorces permet d'éviter l'amplification d'ADN génomique en cas de contamination des échantillons même si aucune contamination n'a été observée sur les courbes de dissociation (ABI, 7900HT). Toutes ces précautions sont dues au fait que la plus petite contamination par de l'ADN génomique peut avoir une répercussion très importante sur les résultats obtenus par une technologie aussi sensible que la RT-PCR quantitative. Un autre critère de choix important est de s'assurer que le couple d'amorces choisi ne forme pas de duplex qui interfèrerait de façon non spécifique avec le produit PCR spécifique obtenu et fausserait ainsi le résultat (inhérent à la technique de SYBR Green utilisée). Enfin, les amorces sont choisies de façon à ce qu'elles ne contiennent pas de régions consensus et/ou polymorphismes. La spécificité totale des séquences nucléotidiques choisies comme amorces du gène cible est testée en réalisant un collage (nucleotide-nucleotide blast) sur l'ensemble du génome humain (Altschul et al., J. Mol. Biol., 215: 403-410, 1990).

• Courbe standard d'amplification

L'efficacité des amorces a été testée par une gamme de dilutions de 5 en 5 (4 points) réalisée en dupliquât sur de l'ARN extrait des cellules NHEK. Seuls les couples d'amorces avec une efficacité proche de 100% (pente égale à 3,32) sont retenus. Une amplification sans matrice (NTC pour Non Template Control) est également réalisée afin de s'assurer qu'il ne se forme pas de duplex pouvant fausser les résultats de PCR quantitative obtenus par la méthode de SYBR Green. La courbe de dissociation obtenue sur l'appareil 7900HT permet de s'assurer que le produit d'amplification obtenu est unique. Un contrôle régulier de la courbe standard d'amplification est réalisé afin de de prévenir une quelconque baisse d'efficacité des amorces.

• Amplification

Les amplifications sont réalisées sur un appareil ABI Prism 7900 Sequence Détection System (Applied Biosystems) par la méthode de SYBR Green (SYBR Green PCR Core Reagents kit, Applied Biosystems). L'amplification est constituée d'une étape de dénaturation (10 min à 95°C) puis par la répétition de 40 cycles d'hybridation (15 sec. à 95°C) et d'extension (1 min. à 65°C) qui assurent une duplication exponentielle de chaque brin.

• Gènes quantifiés

La liste des gènes caractéristiques d'un phénotype de DA qui ont été quantifiés est dans la colonne de gauche dans le Tableau 2. Les valeurs de réponse sont normalisées. De la gauche à la droite du tableau, la colonne DA indique le niveau d'induction de chaque gène (en absence d'actif). La colonne ETOH40, correspond aux valeurs générées par le solvant seul sans actif afin de détecter éventuellement des interférences de réponses dues au solvant. Les 2 colonnes suivantes W01 est l'échantillon à tester à deux concentrations respectives de 1.5 mg/ml et de 0.75 mg/ml après induction. La dernière colonne représente les valeurs générées par le contrôle positif la Dexamethasone à 0.28 mM.

**Résultats**

## TABLEAU 2

| | | DA | EtOH40 | W01 (1,5 mg/ml) | W01 (0,75mg/ml) | DEXA 0,28 mM |
|---|---|---|---|---|---|---|
| **Peptide anti-microbien, immunité innée, récepteur** | | | | | | |
| DEFB103A | Defensin, beta 103B | 27,19 | 11,05 | 498,69 | 32,02 | 107,56 |
| RNASE7 | Ribonuclease, RNase A family, 7 | 31,69 | 27,32 | 117,21 | 13,69 | 43,23 |
| S100A7 | S100 calcium binding protein A7 | 75,22 | 79,10 | 102,11 | 98,50 | 79,12 |
| TLR3 | Toll-like receptor 3 | 15,17 | 15,03 | 0,30 | 0,75 | 2,79 |
| **Interleukines** | | | | | | |
| IFNB1 | Interferon, beta 1, fibroblast | 30,55 | 14,88 | 0,24 | 0,13 | 1,90 |
| IL1A | Interleukin 1, alpha | 56,38 | 41,86 | 19,39 | 7,55 | 8,54 |
| IL1B | Interleukin 1, beta | 67,18 | 33,58 | 4,77 | 3,52 | 2,63 |
| IL4R | Interleukin 4 receptor | 9,63 | 9,33 | 4,35 | 3,14 | 7,03 |
| TSLP | Thymic stromal lymphopoietin | 50,67 | 31,31 | 0,04 | 0,04 | 1,80 |
| **Chemokines** | | | | | | |
| CCL13 | Chemokine (C-C motif) ligand 13 | 3,31 | 2,01 | 0,90 | 0,41 | 1,98 |
| CCL11 | Chemokine (C-C motif) ligand 11 | 0,97 | 0,93 | 0,56 | 0,12 | 1,14 |
| CCL20 | Chemokine (C-C motif) ligand 20 | 85,57 | 55,47 | 0,60 | 0,19 | 28,94 |
| CCL27 | Chemokine (C-C motif) ligand 27 | 16,82 | 12,08 | 0,77 | 2,34 | 0,71 |
| CCL5 | Chemokine (C-C motif) ligand 5 | 78,90 | 99,37 | 2,15 | 2,58 | 49,90 |
| IL15 | Interleukin 15 | 31,54 | 34,53 | 1,44 | 1,50 | 29,16 |
| IL8 | Interleukin 8 | 133,07 | 78,39 | 0,23 | 0,11 | 7,89 |
| CX3CL1 | Fractalkine | 62,38 | 119,24 | 5,90 | 1,83 | 36,63 |

Les résultats représentés sur les tableaux nous permettent de voir le pouvoir d'inhibition des gènes pro-inflammatoires et inflammatoires (Interleukines et Chemokines) et également l'induction de peptides anti-microbiens (ceux-ci sont déficients dans la dermatite atopique). Les peptides anti-microbiens qui sont fortement induits sont: DEFB103, RNASE7, et notamment la psoriasine (S100A7) par W01 de manière dose dépendante. Les interleukines inhibées sont: IFNB1, IL1A et IL1B. Les récepteurs IL4R et TLR3 sont réprimés, le dernier corrèle bien avec l'inhibition quasi-complète du chémokine TSLP. Ces 2 facteurs sont surexprimés chez les patients DA et plus particulièrement dans le cas où apparaît le prurit (Miyagaki et al.2015. J Dermatol Science 78 :89-94). C'est la première fois que nous démontrons une telle inhibition forte anti-TSLP de l'extrait CCV de Mimosa pudica dans ce modèle DA in vitro. Les chémokines CCL11, CCL13, CCL20, CCL27, CCL5, CX3CL1, IL15 et notamment IL8 ont été fortement inhibés par l'extrait W01 quel que soit la dose. En résumé, cette expérience démontre que l'extrait CCV de Mimosa pudica inhibe la majorité des facteurs pro inflammatoires et inflammatoires aussi bien sinon meilleur que le contrôle Dexaméthasone notamment le facteur TSLP cause du prurit chez les patients atteints de DA.

## Revendications

**1.** Procédé de préparation in vitro d'un extrait cellulaire de Mimosa pudica présentant une teneur en mimosine inférieure à 5 ng/g de matière sèche comprenant les étapes suivantes :

- a. Fourniture de matériel végétal stérile de Mimosa pudica,
- b. Dédifférenciation des cellules du matériel végétal,
- c. Mise en culture en suspension des cellules indifférenciées dans un milieu liquide permettant leur maintien dans l'état indifférencié,
- d. Culture de propagation d'une biomasse de cellules indifférenciées dans le milieu de culture,
- e. arrêt de la propagation et obtention d'un extrait cellulaire présentant une teneur en mimosine inférieure à 5 ng/g de matière sèche.

**2.** Procédé selon la revendication 1, **caractérisé en ce que** le matériel végétal de Mimosa pudica est choisi dans le groupe constitué par feuille, tige, pétiole, racine, graine, fleur et bourgeon.

**3.** Procédé selon l'une des revendications 1 ou 2, **caractérisé en ce que** l'étape b) de dédifférenciation est réalisée sur un milieu solide comprenant un ou plusieurs facteurs de croissance.

**4.** Procédé selon l'une des revendications 1 à 3, **caractérisé en ce que** l'étape b) de dédifférenciation est répétée permettant l'obtention de cals de cellules dédifférenciés.

**5.** Procédé selon l'une des revendications 1 à 4, **caractérisé en ce que** l'étape c) est réalisée dans un milieu liquide comprenant un ou plusieurs facteurs de croissance.

**6.** Procédé selon l'une des revendications 1 à 5, **caractérisé en ce que** l'étape d) de culture de propagation est réalisée par des repiquages ou dilutions successifs dans le milieu de culture liquide jusqu'à obtention d'une densité de cellules constante.

**7.** Procédé selon l'une des revendications 1 à 6, **caractérisé en ce qu'**il comprend les étapes supplémentaires suivantes :

- f. séparation liquide/solide,
- g. récupération d'un extrait cellulaire consistant en la biomasse séparée du milieu de culture présentant une teneur en mimosine inférieure à 5 ng/g de matière sèche.

**8.** Procédé selon l'une des revendications 1 à 7, **caractérisé en ce qu'**il comprend une étape supplémentaire de broyage de l'extrait et de récupération d'un broyat de cellules présentant une teneur en mimosine inférieure à 5 ng/g de matière sèche.

**9.** Procédé selon l'une des revendications 1 à 8 **caractérisé en ce que** le milieu de culture de l'étape c) et/ou de l'étape d) comprend un substrat de la phényl-ammonia-lyase.

**10.** Procédé selon la revendication 9, **caractérisé en ce que** l'extrait obtenu contient au moins un acide aminé N-phenylpropenoyl.

**11.** Extrait de culture in vitro de cellules de Mimosa pudica susceptible d'être obtenu par un procédé selon l'une des revendications précédentes présentant une teneur en mimosine inférieure à 5 ng/g de matière sèche.

**12.** Extrait selon la revendication 11 **caractérisé en ce qu'**il contient au moins un acide aminé N-phenylpropenoyl.

**13.** Extrait selon l'une des revendications 11 ou 12, **caractérisé en ce que** les cellules sont des cellules indifférenciées.

**14.** Extrait selon l'une des revendications 11 à 13 pour son utilisation dans le traitement de troubles inflammatoires de la peau.

**15.** Composition dermatologique pour une utilisation pour le traitement d'un trouble inflammatoire de la peau choisi parmi la dermatite atopique, le prurit et démangeaisons dues au prurit, l'eczéma et le psoriasis, comprenant un extrait selon l'une des revendications 11 à 13 en quantité efficace et au moins un excipient dermatologique.

**16.** Utilisation non-thérapeutique d'un extrait selon l'une des revendications 11 à 13 pour le traitement cosmétique du vieillissement cutané et des troubles cutanés associés à un stress oxydatif de la peau dont le stress oxydatif dû à la pollution environnementale.

**17.** Composition cosmétique comprenant un extrait selon l'une des revendications 11 à 13, associé à un excipient cosmétiquement acceptable.

**18.** Composition dermatologique comprenant un extrait selon l'une des revendications 11 à 13, associé à un excipient dermatologiquement acceptable.

**19.** Utilisation non-thérapeutique d'une composition selon la revendication 17 pour le traitement cosmétique du vieillissement cutané et des troubles cutanés associés à un stress oxydatif de la peau, tel que le stress oxydatif dû à la pollution environnementale.

## Patentansprüche

**1.** *In-vitro*-Herstellungsverfahren eines Zellextrakts von *Mimosa pudica,* aufweisend einen Gehalt an Mimosin von unter 5 ng/g Trockenmasse, umfassend die folgenden Schritte:

- a. Bereitstellen von sterilem pflanzlichem Material von *Mimosa pudica,*
- b. Dedifferenzieren der Zellen des pflanzlichen Materials,
- c. Suspensionskultivieren der undifferenzierten Zellen in einem flüssigen Medium, das ihren Verbleib in dem undifferenzierten Zustand erlaubt,
- d. Vermehrungskultivieren einer Biomasse undifferenzierter Zellen in dem Kulturmedium,
- e. Stoppen der Vermehrung und Erhalten eines Zellextrakts, der einen Gehalt an Mimosin von unter 5 ng/g Trockenmasse aufweist.

**2.** Verfahren nach Anspruch 1, **dadurch gekennzeichnet, dass** das pflanzliche Material von *Mimosa pudica* aus der Gruppe ausgewählt ist, die von Blatt, Stil, Blattstil, Wurzel, Samen, Blüte und Knospe gebildet ist.

**3.** Verfahren nach einem der Ansprüche 1 oder 2, **dadurch gekennzeichnet, dass** der Schritt b) der Dedifferenzierung auf einem festen Medium durchgeführt wird, umfassend einen oder mehrere Wachstumsfaktoren.

**4.** Verfahren nach einem der Ansprüche 1 bis 3, **dadurch gekennzeichnet, dass** der Dedifferenzierungsschritt b) wiederholt wird, was den Erhalt von Calli dedifferenzierter Zellen erlaubt.

**5.** Verfahren nach einem der Ansprüche 1 bis 4, **dadurch gekennzeichnet, dass** der Schritt c) in einem flüssigen Medium durchgeführt wird, umfassend einen oder mehrere Wachstumsfaktoren.

**6.** Verfahren nach einem der Ansprüche 1 bis 5, **dadurch gekennzeichnet, dass** der Schritt d) des Vermehrungskultivierens durch aufeinanderfolgendes Pikieren oder Verdünnungen im flüssigen Kulturmedium bis zum Erhalt einer

konstanten Zelldichte durchgeführt wird.

7. Verfahren nach einem der Ansprüche 1 bis 6, **dadurch gekennzeichnet, dass** es die folgenden zusätzlichen Schritte umfasst:

   - f. Trennen Flüssigkeit/Feststoff,
   - g. Rückgewinnen eines Zellextrakts, das aus der vom Kulturmedium getrennten Biomasse besteht, aufweisend einen Gehalt an Mimosin von unter 5 ng/g Trockenmasse.

8. Verfahren nach einem der Ansprüche 1 bis 7, **dadurch gekennzeichnet, dass** es einen zusätzlichen Schritt des Zerkleinerns des Extrakts und des Rückgewinnens eines Zellzerkleinerungsprodukts umfasst, aufweisend einen Gehalt an Mimosin von unter 5 ng/g Trockenmasse.

9. Verfahren nach einem der Ansprüche 1 bis 8, **dadurch gekennzeichnet, dass** das Kulturmedium von Schritt c) und/oder von Schritt d) ein Phenyl-Ammonia-Lyase-Substrat umfasst.

10. Verfahren nach Anspruch 9, **dadurch gekennzeichnet, dass** der erhaltene Extrakt mindestens eine N-Phenylpropenoyl-Aminosäure enthält.

11. *In-vitro*-Kulturextrakt von Zellen von *Mimosa pudica,* die durch ein Verfahren nach einem der vorangehenden Ansprüche gewinnbar sind, aufweisend einen Gehalt an Mimosin von unter 5 ng/g Trockenmasse.

12. Extrakt nach Anspruch 11, **dadurch gekennzeichnet, dass** er mindestens eine N-Phenylpropenoyl-Aminosäure enthält.

13. Extrakt nach einem der Ansprüche 11 oder 12, **dadurch gekennzeichnet, dass** die Zellen undifferenzierte Zellen sind.

14. Extrakt nach einem der Ansprüche 11 bis 13 für seine Verwendung bei der Behandlung von entzündlichen Störungen der Haut.

15. Dermatologische Zusammensetzung für eine Verwendung zur Behandlung einer entzündlichen Störung der Haut, ausgewählt aus der atopischen Dematitis, dem Pruritus und dem von Pruritus ausgelösten Juckreiz, dem Ekzem und der Psoriasis, umfassend einen Extrakt nach einem der Ansprüche 11 bis 13 in wirksamer Menge und mindestens einen dermatologischen Hilfsstoff.

16. Nichttherapeutische Verwendung eines Extrakts nach einem der Ansprüche 11 bis 13 zur kosmetischen Behandlung der Hautalterung und der Hautstörungen in Verbindung mit einem oxidativen Stress der Haut, darunter der oxidative Stress aufgrund von Umweltverschmutzung.

17. Kosmetische Zusammensetzung, umfassend einen Extrakt nach einem der Ansprüche 11 bis 13 in Verbindung mit einem kosmetisch akzeptablen Hilfsstoff.

18. Kosmetische Zusammensetzung, umfassend einen Extrakt nach einem der Ansprüche 11 bis 13 in Verbindung mit einem dermatologisch akzeptablen Hilfsstoff.

19. Nichttherapeutische Verwendung einer Zusammensetzung nach Anspruch 17 zur kosmetischen Behandlung der Hautalterung und der Hautstörungen in Verbindung mit einem oxidativen Stress der Haut wie der oxidative Stress aufgrund von Umweltverschmutzung.

## Claims

1. A process for *in vitro* preparation of a *Mimosa pudica* cell extract having a mimosine content of less than 5 ng/g dry weight, comprising the following steps:

   - a. Provision of sterile plant material of *Mimosa pudica,*
   - b. Differentiation of cells from the plant material,

- c. Suspension culture of the undifferentiated cells in a liquid medium for maintaining them in the undifferentiated state,
- d. Propagation culture of an undifferentiated cell biomass in the culture medium,
- e. Stopping the propagation and obtaining a cell extract having a mimosine content of less than 5 ng/g dry weight.

**2.** The process according to claim 1, **characterized in that** the *Mimosa pudica* plant material is selected from the group consisting of leaf, stem, petiole, root, seed, flower and bud.

**3.** The process according to one of claims 1 or 2, **characterized in that** step b) of dedifferentiation is carried out on a solid medium containing one or more growth factors.

**4.** The process according to one of claims 1 to 3, **characterized in that** step b) of dedifferentiation is repeated so as to obtain calluses of dedifferentiated cells.

**5.** The process according to one of claims 1 to 4, **characterized in that** step c) is carried out in a liquid medium containing one or more growth factors.

**6.** The process according to one of claims 1 to 5, **characterized in that** step d) of propagation culture is carried out by successive subcultures or dilutions in the liquid culture medium until a constant cell density is obtained.

**7.** The process according to one of claims 1 to 6, **characterized in that** it comprises the following additional steps:

- f. liquid/solid separation,
- g. recovery of a cell extract consisting of biomass separated from the culture medium having a mimosine content of less than 5 ng/g dry weight.

**8.** The process according to one of claims 1 to 7, **characterized in that** it comprises an additional step of crushing the extract and recovering a crushed cell material having a mimosine content of less than 5 ng/g dry weight.

**9.** The process according to one of claims 1 to 8, **characterized in that** the culture medium of step c) and/or step d) contains a phenyl-ammonia-lyase substrate.

**10.** The process according to claim 9, **characterized in that** the resulting extract contains at least one N-phenylpropenoyl amino acid.

**11.** An *in vitro* culture extract of *Mimosa pudica* cells obtainable by a process according to one of the preceding claims having a mimosine content of less than 5 ng/g dry weight.

**12.** The extract according to claim 11 **characterized in that** it contains at least one N-phenylpropenoyl amino acid.

**13.** The extract according to one of claims 11 or 12, **characterized in that** the cells are undifferentiated cells.

**14.** The extract according to one of claims 11 to 13 for use in the treatment of inflammatory skin disorders.

**15.** A dermatological composition for use in the treatment of an inflammatory skin disorder selected from atopic dermatitis, pruritus and itching due to pruritus, eczema and psoriasis, comprising an extract according to one of claims 11 to 13 in an effective amount and at least one dermatological excipient.

**16.** Non-therapeutic use of an extract according to one of claims 11 to 13 for the cosmetic treatment of skin aging and of skin disorders associated with skin oxidative stress, including oxidative stress due to environmental pollution.

**17.** A cosmetic composition comprising an extract according to one of claims 11 to 13 combined with a cosmetically acceptable excipient.

**18.** A dermatological composition comprising an extract according to one of claims 11 to 13 combined with a dermatologically acceptable excipient.

**19.** Non-therapeutic use of a composition according to claim 17 for the cosmetic treatment of skin aging and of skin

disorders associated with skin oxidative stress, such as oxidative stress due to environmental pollution.

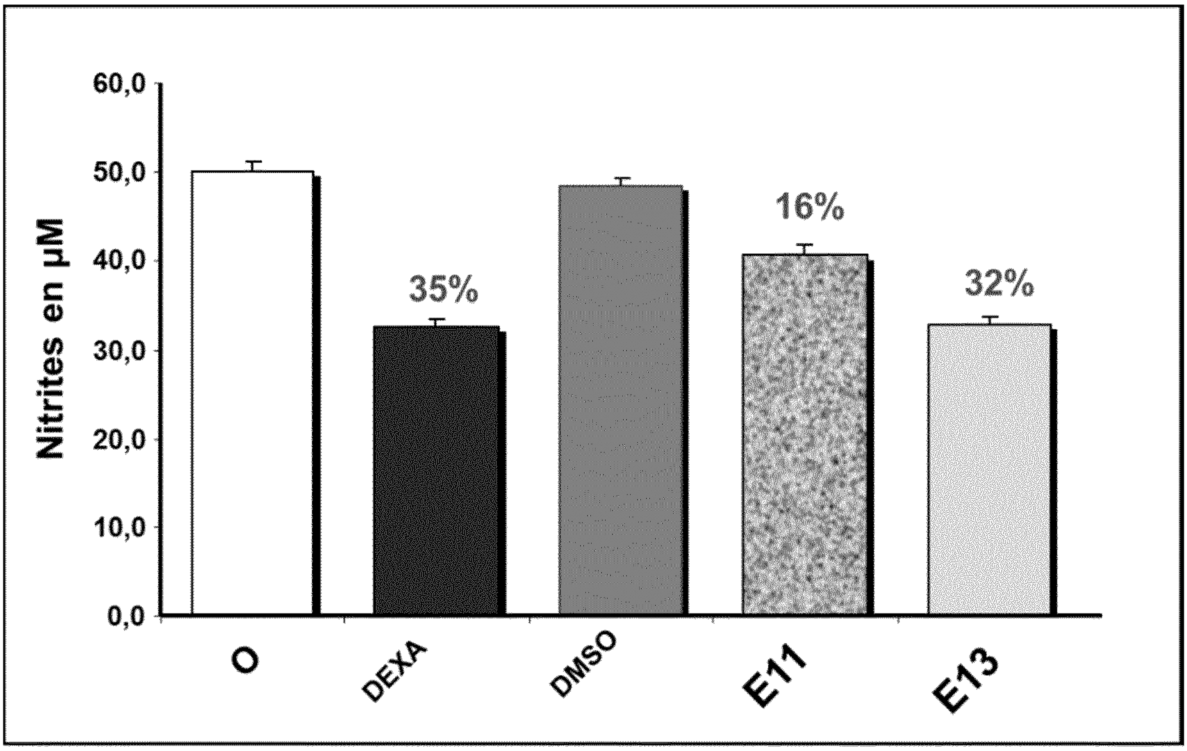
60,0
50,0
40,0
30,0
20,0
10,0
0,0
Nitrites en µM
35%
16%
32%
O
DEXA
DMSO
E11
E13

Figure 1

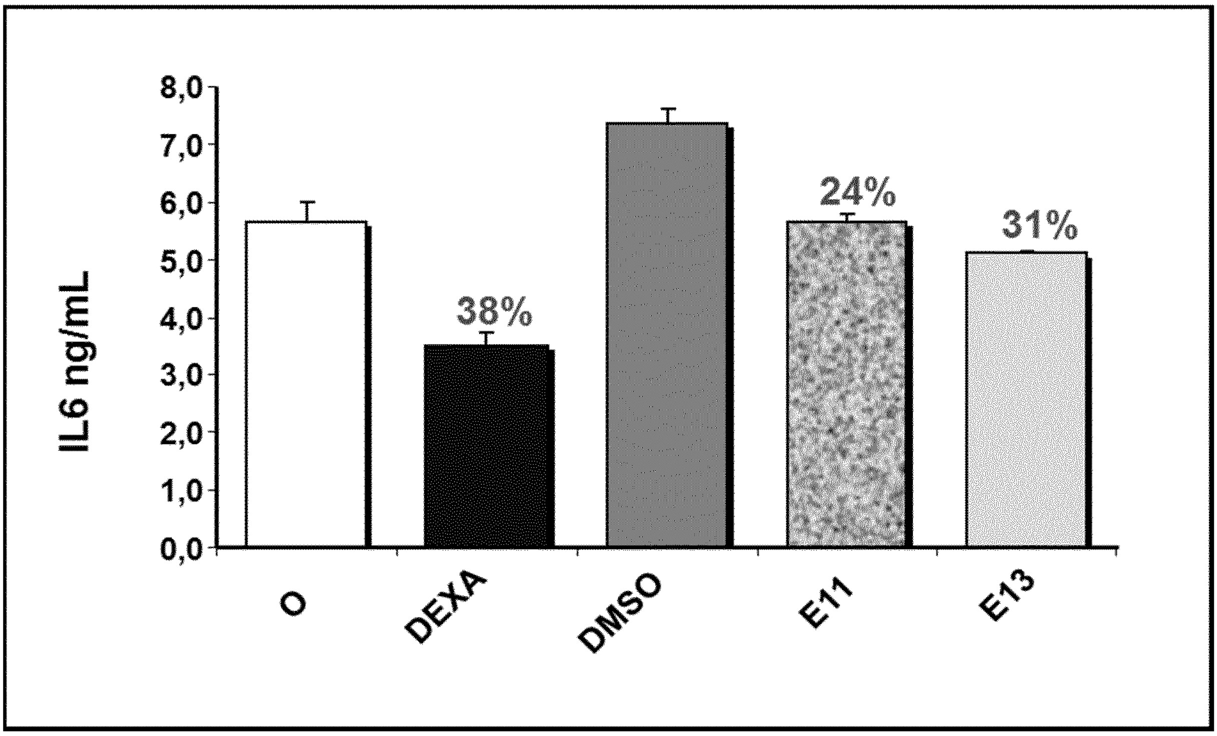


Figure 2

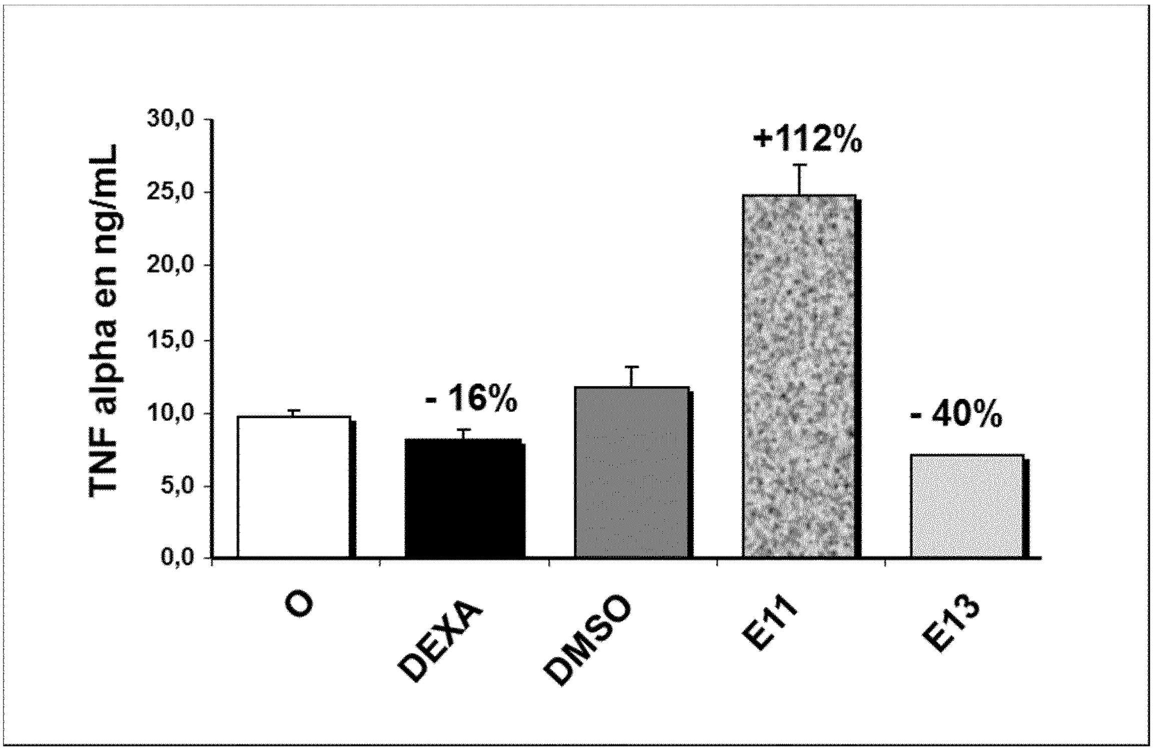
TNF alpha en ng/mL
30,0
25,0
20,0
15,0
10,0
5,0
0,0
+112%
- 16%
- 40%
O
DEXA
DMSO
E11
E13

Figure 3

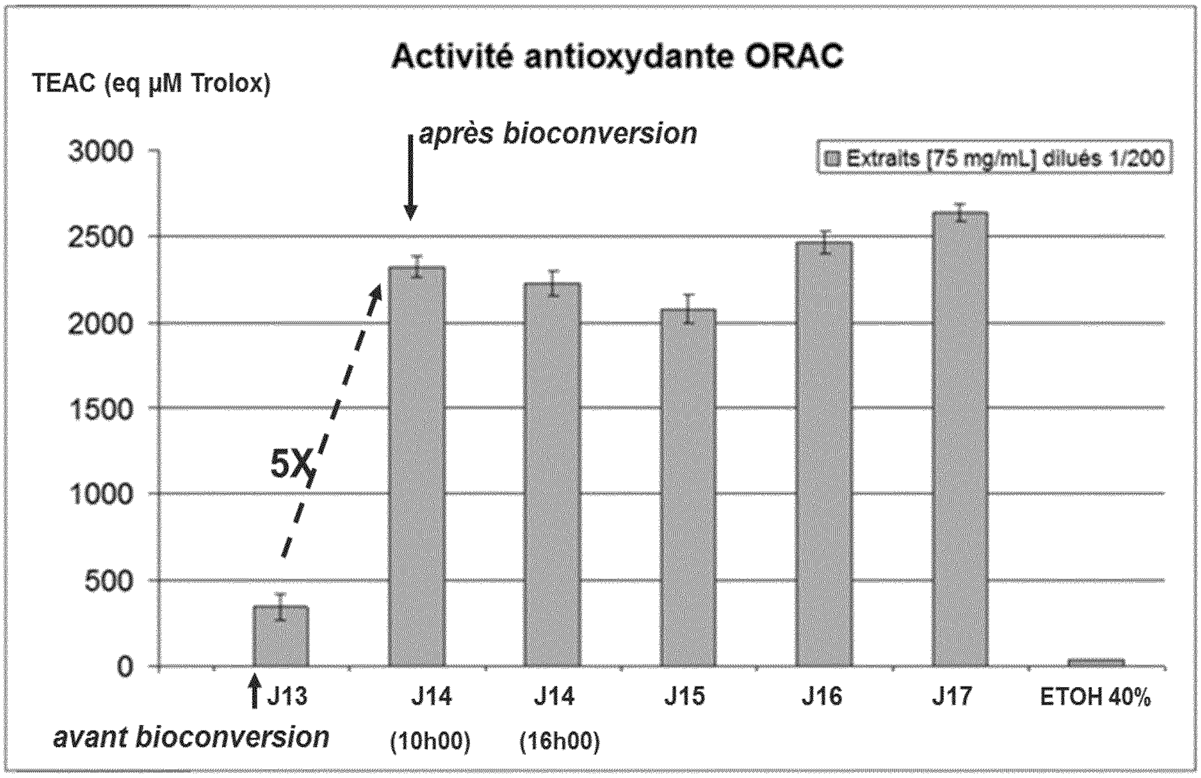
Activité antioxydante ORAC
TEAC (eq µM Trolox)
après bioconversion
Extraits [75 mg/mL] dilués 1/200
3000
2500
2000
1500
1000
500
0
5X
J13
J14
J14
J15
J16
J17
ETOH 40%
avant bioconversion
(10h00)
(16h00)

Figure 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- KR 101064848


### Littérature non-brevet citée dans la description


- **PAUL et al.** *Int J Bio Med Res,* 2010, vol. 1 (4), 223-227
- **ZHANG et al.** *Pharmacogn. Mag,* 2011, vol. 4, 35-39
- **KULP K.S.** *Toxicology and applied pharmacology,* 1996, vol. 139, 356-364
- **WILLIAMS RD et al.** *Allelopathy J.,* 2007, vol. 19 (2), 423-430
- **HENSEL et al.** *Planta Med.,* 2007, vol. 73, 142-150
- **ZENG et al.** *J. Agric. Food Chem.,* 2011, vol. 59, 5342-5350
- **TAKAI et al.** *Allergology Int.,* 2012, vol. 61, 3-17
- **MURASHIGE, T. ; SKOOG, F.** A revised medium for rapid growth and bio assays with tobacco tissue cultures. *Physiol. Plant,* 1962, vol. 15, 473-496
- Plant Culture Media. **E. F. GEORGE ; D. J. M. PUTTOCK ; H. J. GEORGE.** Formulations and Uses. Exegetics Ltd, 1987, vol. 1
- **KANG et al.** *J Pharmacol Exp Ther.,* 2002, vol. 302, 138-144
- **DÉVALOS A. et al.** *Polish journal of food and nutrition sciences,* 2003, vol. 12/53, 133-136
- **CASTEX-RIZZI.** *Br J Dermatol.,* 2014, vol. 170 (1), 12-8
- **ALTSCHUL et al.** *J. Mol. Biol.,* 1990, vol. 215, 403-410
- **MIYAGAKI et al.** *J Dermatol Science,* 2015, vol. 78, 89-94